Europäisches Patentamt

European Patent Office

Office européen des brevets

(19)

(11) Publication number: 0 139 501

A2

(12) **EUROPEAN PATENT APPLICATION**

(21) Application number: 84306767.9

(22) Date of filing: 04.10.84

(51) Int. Cl.⁴: **C 12 N 15/00**
C 12 P 21/02, C 07 H 21/04
C 12 N 1/18, C 12 N 1/20
//(C12P21/02, C12R1:19, 1:865)

(30) Priority: 11.10.83 US 540634

(43) Date of publication of application:
02.05.85 Bulletin 85/18

(84) Designated Contracting States:
AT BE CH DE FR GB IT LI LU NL SE

(71) Applicant: BEATRICE COMPANIES, INC.
Two North LaSalle Street
Chicago Illinois 60602(US)

(72) Inventor: Date, Takayasu Department of Biological
Chemistry
Kanazawa Medical University Uchinada-cho
Ishikawa-ku 92002 Kanazawa-shi(JP)

(72) Inventor: Grunstein, Michael
1020 Chantilly
Los Angeles California 90064(US)

(72) Inventor: Hollenberg, Stanley
7894 Caminito Jonata
San Diego California 92122(US)

(72) Inventor: Koduri, Raju
3745 Vinton Avenue
Los Angeles California 90034(US)

(72) Inventor: Lai, Jiunu
279 South Atlantic Boulevard
Monterrey Park California 91754(US)

(72) Inventor: Lee, Jar-How
2840 Glendon Avenue
Torrance California 90504(US)

(72) Inventor: Wilcox, Gary
3637 Seahorn Drive
Malibu California 90265(US)

(74) Representative: Brown, John David et al,
FORRESTER & BOEHMERT Widenmayerstrasse 4/I
D-8000 München 22(DE)

(54) The manufacture and expression of genes for thaumatin.

(57) Disclosed are DNA sequences comprising structural genes coding for a polypeptide having the amino acid sequence and having one or more of the biochemical or immunological properties of thaumatin. Structural gene sequences may be provided with initial and terminal sequences which facilitate production of discrete protein products (.eg., thaumatin I) by selected host microorganisms (e.g., *E. coli* and yeast) as well as for expression by host organisms of fusion proteins (e.g., L-ribulokinase-thaumatin I) from which the desired products may be isolated.

EP 0 139 501 A2

"THE MANUFACTURE AND EXPRESSION OF GENES FOR THAUMATIN"

BACKGROUND

The present invention relates generally to the manipulation of genetic materials and more particularly to the manufacture of specific DNA sequences useful in recombinant procedures to secure the production of a polypeptide having one or more of the biochemical or immunological properties of thaumatin.

Thaumatin is an extremely sweet-tasting protein produced in the arils of the fruit of the African shrub Thaumatococcus danielli Benth. The fruit traditionally has been used in West Africa as a sweetener of palm wine, corn, bread, and sour fruit. Thaumatin, which is about 1600 times sweeter than sucrose on a weight basis, is produced in at least five forms: thaumatin I, II, a, b and c. These proteins, named in their order of elution from an ion exchange column [Higgenbotham, et al., in Sensory Properties of Foods (Birch, et al., eds.), London: Applied Sciences, pp. 129-149 (1977)], have molecular weights of approximately 22 kilodaltons. Thaumatin I and II are nearly identical proteins, each consisting of a single unmodified polypeptide chain, 207 amino acid residues in length, differing in only five amino acids.

- 2 -

0139501

Thaumatin I and II are non-toxic proteins, are low-calorie and non-cariogenic, and elicit profound taste responses suggesting a stable interaction between these proteins and human taste buds. Therefore, thaumatin has potential for use as a sugar substitute, food additive, a sweetness receptor probe and a tool for further elucidation of the taste response.

A plentiful supply of pure thaumatin is required to utilize the protein as a possible food additive and research tool. Because the thaumatin plant requires a tropical climate and insect pollination for successful fruit propagation, there are considerable difficulties involved in greenhouse cultivation of the fruit.

The entire amino acid sequence of thaumatin I has been determined directly and is set out below [Iyengar, et al., Eur.J.Biochem., 96, 193-204 (1979)].

1                                          10
NH$_2$-Ala-Thr-Phe-Glu-Ile-Val-Asn-Arg-Cys-Ser-Tyr-Thr-Val-

20
Trp-Ala-Ala-Ala-Ser-Lys-Gly-Asp-Ala-Ala-Leu-Asp-Ala-Gly-

30                                         40
Gly-Arg-Gln-Leu-Asn-Ser-Gly-Glu-Ser-Trp-Thr-Ile-Asn-Val-

50
Glu-Pro-Gly-Thr-Asn-Gly-Gly-Lys-Ile-Trp-Ala-Arg-Thr-Asp-

60
Cys-Tyr-Phe-Asp-Asp-Ser-Gly-Ser-Gly-Ile-Cys-Lys-Thr-Gly-

70                                         80
Asp-Cys-Gly-Gly-Leu-Leu-Arg-Cys-Lys-Arg-Phe-Gly-Arg-Pro-

90
Pro-Thr-Thr-Leu-Ala-Glu-Phe-Ser-Leu-Asn-Gln-Tyr-Gly-Lys-

100                                        110
Asp-Tyr-Ile-Asp-Ile-Ser-Asn-Ile-Lys-Gly-Phe-Asn-Val-Pro-

120
Met-Asn-Phe-Ser-Pro-Thr-Thr-Arg-Gly-Cys-Arg-Gly-Val-Arg-

130
Cys-Ala-Ala-Asp-Ile-Val-Gly-Gln-Cys-Pro-Ala-Lys-Leu-Lys-

```
140                                              150
Ala-Pro-Gly-Gly-Gly-Cys-Asn-Asp-Ala-Cys-Thr-Val-Phe-Gln-
                      160
Thr-Ser-Glu-Tyr-Cys-Cys-Thr-Thr-Gly-Lys-Cys-Gly-Pro-Thr-
         170                                     180
Glu-Tyr-Ser-Arg-Phe-Phe-Lys-Arg-Leu-Cys-Pro-Asp-Ala-Phe-
                                190
Ser-Tyr-Val-Leu-Asp-Lys-Pro-Thr-Thr-Val-Thr-Cys-Pro-Gly-
              200                          207
Ser-Ser-Asn-Tyr-Arg-Val-Thr-Phe-Cys-Pro-Thr-Ala-COOH
```

The amino acid sequence for thaumatin II has been deduced from its nucleotide sequence [Edens, et al., Gene, 18, 1-12 (1982)] and a gene for thaumatin II has been cloned from messenger RNA-derived cDNA. The five amino acids in the thaumatin II sequence which differ from the thaumatin I sequence above are the following: lysine instead of asparagine at residue 46; arginine instead of serine at residue 63; arginine instead of lysine at residue 67; glutamine instead of arginine at residue 76; and aspartic acid instead of asparagine at residue 113. Sequence analysis also indicated that thaumatin II is initially translated as a precursor form, preprothaumatin, with both a 22 residue amino-terminal extension and an acidic, six-amino acid carboxy terminal tail. The amino terminal peptide was postulated as a secretion signal based on its hydrophobic character and a compartmentalization role was hypothesized for the carboxy terminal extension.

The Edens, et al. reference cited above notes that a polypeptide having the native sequence of preprothaumatin II has been microbially produced. More specifically, the reference and European Patent Application Nos. 54,330 and 54,331 disclose cDNA sequences coding for native mature thaumatin II and preprothaumatin II and also disclose cloning vehicles comprising the DNA sequences for use in transformation in microorganisms.

Genes isolated by the cDNA method such as described in European Patent Application Nos. 54,330 and 54,331 have a number of disadvantages. Restriction enzyme sites occur randomly and may not allow further manipulations of the gene such as are involved in vector construction. Generation of cDNA clones by the method of Villa-Komaroff, et al., [PNAS-USA, 75, 3727-3731 (1978)] introduces a homopolymer of GC base pairs upstream from the gene, which may cause difficulty in attaining efficient production of the polypeptide. Host microorganisms seldom possess the physiological apparatus for processing protein products in the same way as cells from which the DNA is derived. In the case of cDNA genes coding for precursor polypeptides like preprothaumatin, it is necessary to remove initial and terminal DNA sequences and introduce microbial transcription initiating and translation terminating codons if the protein is to be produced in its mature form.

The most important problem encountered by employing cDNA methods may be the incompatibility between the codons extant in the natural gene and those commonly preferred by the expression host. This incompatibility can prevent efficient translation of mRNA in the host. This may especially be true in the case of thaumatin, where a comparison of the codons extant in the thaumatin II gene [Edens, et al., supra] and those apparently preferred by yeast [Bennetzen, et al., J.Biol.Chem., 257, 3026-3031 (1982)] and E.coli [Grosjean, et al., Gene, 18, 199-209 (1982)] show a marked divergence.

The above-noted difficulties inherent in the use of cDNA-derived genes for microbial expression of desired polypeptides can be avoided by direct chemical synthesis of the gene. While total synthesis of large structural genes has been reported occasionally [see, Edge, et al., Nature, 292, 756-762 (1981)], synthetic methods have not yet been brought to bear in the question of genes coding for thaumatin I and thaumatin II.

## BRIEF SUMMARY

Provided by the present invention are manufactured genes capable of directing synthesis in a selected host microorganism of a polypeptide having one or more of the biochemical or immunological properties of thaumatin, e.g., thaumatin I and II. In preferred forms of manufactured genes, the base sequences include one or more codons selected from among alternative codons specifying the same amino acid on the basis of preferential expression characteristics of the codon in a projected host microorganism, e.g., E.coli, S.cerevisiae. Other preferred forms of manufactured genes include those wherein: (1) a codon specifies an additional amino acid in the polypeptide synthesized which facilitates direct expression in E.coli organisms (e.g., an initial methionine residue) and/or yeast organisms; and/or (2) codons specifying thaumatin polypeptides are preceded and/or followed by a sequence of bases comprising a portion of a base sequence which provides for restriction endonuclease cleavage of a DNA sequence (e.g., a BamHI site) and consequently facilitates formation of expression vectors.

Also provided by the present invention are fusion genes comprising manufactured genes according to the invention fused to a second gene capable of directing synthesis of a second polypeptide (e.g., L-ribulokinase) in a manner permitting the synthesis of a fused polypeptide including a thaumatin polypeptide.

In practice of the invention to generate polypeptide products, DNA sequences including manufactured genes are inserted into a viral or circular plasmid DNA

- 6 -

vector to form a hybrid vector and the hybrid vectors are employed to transform host microorganisms such as bacteria (e.g., E.coli) or yeast cells (e.g., S.cerevisiae). Vectors may also be supplied with appropriate promoter/regulator DNA sequences, allowing for controlled expression in the host microorganism. The transformed microorganisms are thereafter grown under appropriate nutrient conditions and express the polypeptide products of the invention.

As a further aspect of the invention, there are provided improved processes for selectively altering the nucleotide sequence of a plasmid DNA sequence having, respectively, at least two unique restriction or at least three unique restriction endonuclease recognition sites, by employing a primer oligonucleotide containing a selected alteration. The novel processes were developed in the course of developing the manufactured gene of the present invention.

Other aspects and advantages of the present invention will be apparent upon consideration of the following detailed description thereof.

## DETAILED DESCRIPTION

As employed herein, the term "manufactured" as applied to a DNA sequence or gene shall designate a product either totally chemically and enzymatically synthesized by assembly of nucleotide bases or derived from the biological replication of a product thus synthesized. As such, the term is exclusive of products "synthesized" by cDNA methods or genomic cloning methodologies which involve starting materials which are initially of biological origin.

The following abbreviations shall be employed herein to designate amino acids: Alanine, Ala; Arginine, Arg; Asparagine, Asn; Aspartic acid, Asp; Cysteine, Cys;

Glutamine, Gln; Glutamic acid, Glu; Glycine, Gly; Histidine, His; Isoleucine, Ile; Leucine, Leu; Lysine, Lys; Methionine, Met; Phenylalanine, Phe; Proline, Pro; Serine, Ser; Threonine, Thr; Tryptophan, Trp; Tyrosine, Tyr; Valine, Val. The following abbreviations shall be employed for nucleotide bases: A for adenine; G for guanine; T for thymine; U for uracil; and C for cytosine.

For ease of understanding of the present invention, Table I below provides a tabular correlation between the 64 alternate triplet nucleotide base codons of DNA and the 20 amino acids and transcription termination ("stop") function specified thereby.

TABLE I

| FIRST POSITION | SECOND POSITION | | | | THIRD POSITION |
|---|---|---|---|---|---|
| | T | C | A | G | |
| T | Phe | Ser | Tyr | Cys | T |
| | Phe | Ser | Tyr | Cys | C |
| | Leu | Ser | Stop | Stop | A |
| | Leu | Ser | Stop | Trp | G |
| C | Leu | Pro | His | Arg | T |
| | Leu | Pro | His | Arg | C |
| | Leu | Pro | Gln | Arg | A |
| | Leu | Pro | Gln | Arg | G |
| A | Ile | Thr | Asn | Ser | T |
| | Ile | Thr | Asn | Ser | C |
| | Ile | Thr | Lys | Arg | A |
| | Met | Thr | Lys | Arg | G |
| G | Val | Ala | Asp | Gly | T |
| | Val | Ala | Asp | Gly | C |
| | Val | Ala | Glu | Gly | A |
| | Val | Ala | Glu | Gly | G |

The following example illustrates a preferred general procedure for preparation and assembly of deoxyoligonucleotides for use in the manufacture of DNA sequences of the invention.

## EXAMPLE 1

An entire synthetic gene for thaumatin I was assembled through use of 28 principal oligonucleotide fragments (designated 1a, 1b, 2a, 2b, etc., in Table II, below) having a length of 25 to 39 residues and designed to encode the amino acid sequence of thaumatin I published by Iyengar, et al., supra. The gene contained codons corresponding to the "favored triplets" of S.cerevisiae based on the Bennetzen, et al., supra, study of highly expressed yeast gene sequences.

## TABLE II

1a  5'-(CCAG) TGATC ATG GCT ACC TTC GAA ATC GTT A-3'

1b  3'-CTT TAG CAA TTG TCT ACA AGA ATG TGA CAA A-5'

2a  5'-GG GCT GCT GCT TCC AAG GGT GAC GCT G-3'

2b  3'CA CTG CGA CGA AAC CTG C**T̈C̈G̈Ä̈G**(TTCC)-5'

3a  5'-(CCTT)G**Ä̈G̈C̈T̈C** C GGT GGT AGA CAA TTG A-3'

3b  3'-CT GTT AAC TTG AGA CCA CTT AGG AC-5'

4a  5'-G ACC ATC AAC GTC GAA CCA GGT AAC AA-3'

4b  3'-T CCA TTG TTG CCA CCA TTC TAG A (TTCC)-5'

5a  5'-(CCTT) AG ATC TGG GCT AGA ACC GAC TGT TAC TTC GAT G-3'

5b  3'-TG AAG CTA CTG AGA CCA AGG CCA TAG ACA TTC TGA CC-5'

6a  5'-T GAC TGT GGT GGT TTG TTG AGA TGT AAG AGA TTC GGT-3'

6b  3'-TCT AAG CCA TCT GGT GGT TGG TGA AAC CGA CTT AAG (TCT)-5'

```
7a    5'-(GCT) GAA TTC TCT TTG AAC CAA TAC GGT AAG GAC-3'
7b    3'-CCA TTC CTG ATG TAG CTA TAG AGG TTG TAG TTC-5'
8a    5'-GGT TTC AAC GTT CCA ATG AAC TTC TCT CCA ACC-3'
                                             ****
8b    3'-AGA GGT TGG GCA TCT CCA ACA TCT CCTCGAG(TTCC)-5'
         ****
9a    5'-(TTCC)GAGCTC GTC AGA TGT GCT GCT GAC ATC-3'
9b    3'-CTG TAG CAA CCA GTT ACA GGT CGA TTC GAA
         (CCCTT)-5'
10a   5'-(TTCCT) AAG CTT AAG GCT CCA GGT GGT GGT TGT
         AAC-3'
10b   3'-CCA ACA TTG CTG CGA ACA TGG CAA AAG GTT TGA-5'
11a   5'-TCC GAA TAC TGT TGT ACC ACT GGT AAG TGT GGT-3'
11b   3'-TTC ACA CCA GGT TGG CTT ATC AGA TCT (CCTT)-5'
12a   5'-(TTCC) TCT AGA TTC TTC AAG AGA TTG TG-3'
                                 ****
12b   3'-C TCT AAC ACA GGT CTG CTCGAG(TTC)-5'
         ****
13a   5'-(TTCC)GAGCTC T TTC TCC TAC GTC TTG GAC AA-3'
13b   3'-G AAC CTG TTC GGT TGA TGG CAG TGA ACA GGT-5'
14a   5'-GGT TCT TCC AAC TAC AGA GTT ACC TTC TCT C-3'
14b   3'-GG AAG ACA GGT TGA CGG ATT ACT GAG CTC (CCTT)-5'
```

As often as possible, codons were employed to introduce unique restriction enzyme recognition sites at regular intervals into the sequence. In two cases, rarely used codons were required to form recognition sites at desired locations, i.e., the codon for glycine at residue 123 in Table VI, infra, is GGC, rather than the "favored triplet" GGT, and the codon for leucine at residue 138 is CTT, rather than the "favored triplet" TTG. As an aid to assembly of the entire gene from intermediate double-stranded sequences, oligonucleotides were designed to allow for the presence in intermediate structures of three SstI sites within (and interruptive of) the protein coding region. The "extra" bases needed to develop these sites are within parentheses and noted by asterisks in Table II. As discussed infra, these bases are deleted from the gene in the course of final assembly. Additional bases within parentheses in Table II are included to insure efficient digestion of duplexes formed and are not part of the intermediate one-pair or two-pair segments or the final gene.

The 28 oligonucleotides corresponding to regions of the thaumatin I sequence were synthesized by the phosphotriester method on a solid support, with dimer couplings, and purified by high performance liquid chromatography according to Ito, et al., Nucleic Acids Res., 10, 1755-1769 (1982). In some cases the Ito, et al. process was modified to include initiating the assembly of each oligonucleotide from 30 mg nucleoside-bound (0.10-0.17 mmole/g) polystyrene resin (1% crosslinked) rather than the usual 50-60 mg resin. The average yield of oligonucleotide per dimer coupling was 90% to 99%.

The following example illustrates the manipulations performed on the oligonucleotides to assemble them into duplexes and combine duplexes into gene segments for cloning into a vector.

### EXAMPLE 2

As summarized in Table III, below, the 28 oligonucleotides synthesized in Example 1 were assembled into partial duplexes containing a sense and antisense strand with a 9 or 10 base pair overlap at the 3' terminus of each oligonucleotide.

TABLE III

| Oligo-nucleotides Combined | Oligo-nucleotide 5' End-Labeled | Restriction Endonuclease | "c" Sequence Employed |
|---|---|---|---|
| 1a and 1b | 1b | BclI | ---- |
| 2a and 2b | 2a | SstI | ---- |
| 3a and 3b | 3b | SstI | ---- |
| 4a and 4b | 4a | BglII | ---- |
| 5a and 5b | 5b | BglII | ---- |
| 6a and 6b | 6a | EcoRI | ---- |
| 7a and 7b | 7b | EcoRI | ---- |
| 8a and 8b | 8a | SstI | 5'-ACTAGAGGTTGTAGA-3' |
| 9a and 9b | None | SstI HindIII | 5'-AGCAGCAGATCTGACGA-3' |
| 10a and 10b | 10b | HindIII | ---- |
| 11a and 11b | 11a | XbaI | ---- |
| 12a and 12b | None | XbaI SstI | ---- |
| 13a and 13b | 13b | SstI | 5'-GCCAACTACCGTCACTT-3' |
| 14a and 14b | 14a | XhoI | 5'-CAACTGCCTATTGACTCGA-3' |

0139501

The oligonucleotide of each pair which was to participate in a blunt-end ligation was 5'-end labeled. Labeling was performed with [$^{32}$P] transfer from [$\gamma$-$^{32}$P]ATP by T4 polynucleotide kinase according to the procedure of Sgaramella, et al., J.Mol.Bio., 72, 427-444 (1972) and complete phosphorylation was effected by subsequent ATP (0.5 mm) addition. All oligonucleotide pairs were labeled in this fashion except oligonucleotide pairs 9 and 12. These oligonucleotide pairs were subsequently to be digested at both ends, so prior 5' phosphorylation would not facilitate visualization of a doubly-digested duplex. For these oligonucleotide pairs radiolabeling was performed with [$\alpha$-$^{32}$P]dATP during the elongation.

The labeled oligonucleotide was mixed with an equal amount of its unlabeled partner (50-200 ng), heated to 90°C for one minute and slowly cooled to 23°C to allow proper annealing. Oligonucleotides were extended at 23°C for one hour with one unit DNA polymerase I (Klenow), or 24 units reverse transcriptase and 0.5 mM deoxynucleoside triphosphates. The enzyme was subsequently denatured and the mixture desalted.

Each oligonucleotide pair was digested overnight with the appropriate restriction endonuclease and the enzyme denatured. Analysis of the digestion mixture was carried out by autoradiography of a nondenaturing 15% polyacrylamide gel and the desired band excised. This fragment was eluted from the gel by diffusion at 37°C overnight and concentrated and further purified by passage through a BND-cellulose mini-column [Rossi, J., et al., J.Mol.Biol., 128, 21-47 (1979)].

For oligonucleotide pairs 8a plus 8b, 9a plus 9b, 13a plus 13b, and 14a plus 14b, self-annealing of fragments was observed. Therefore, to direct the proper annealing, a third "c" oligonucleotide was synthesized which was complementary to a region of one of the oligonucleotides and contiguous with the overlap region. This "c" oligonucleotide acted as an extension of the overlap region and was elongated to produce the desired duplex.

In cases where a "c" oligonucleotide was required, the above protocol with several modifications was followed. The scale for duplex synthesis was increased due to the lower yields. 250 ng to 1 μg of each oligonucleotide (a and b) was used and a two- to six-fold molar excess of the phosphorylated "c" oligonucleotide was added. The three oligonucleotides were mixed after labeling and phosphorylation of the appropriate oligonucleotide(s), denatured, annealed and extended as before. After restriction enzyme digestion the duplex was purified by 15% polyacrylamide gel electrophoresis, but at a very low current to prevent melting of the extended "c" oligonucleotide from the duplex. After digestion this oligonucleotide is bound to the duplex by the hydrogen bonding of 15 base pairs (duplexes 9 and 14), 17 base pairs (duplex 8) or 23 base pairs (duplex 13). Polymerization of approximately 30% of the labeled oligonucleotide was achieved thereby.

As indicated in Table IV, below, extended and digested duplexes 9 (from oligonucleotides 9a and 9b) and 12 (from oligonucleotides 12a and 12b) forming a one-duplex segment had restriction endonuclease enzyme sticky ends at both termini. The extended duplexes designed to form a two-duplex segment (duplexes 1 and 2, 3 and 4, 5 and 6, 7 and 8, 10 and 11, 13 and 14) had a sticky end at one terminus and a blunt end at the other.

TABLE IV

Pair Sequence
BclI

1    5'-GATC ATG GCT ACC TTC GAA ATC GTT AAC AGA TGT TCT TAC ACT GTT T-3'
         3'--TAC CGA TGG AAG CTT TAG CAA TTG TCT ACA AGA ATG TGA CAA A-5'

                                                                     SstI

2    5'-GG GCT GCT GCT TCC AAG GGT GAC GCT GCT TTG GAC GAGCT-3'
     3'-CC CGA CGA CGA AGG TTC CCA CTG CGA CGA AAC CTG C-5'

         SstI

3      5'-CC GGT GGT AGA CAA TTG AAC TCT GGT GAA TCC TG-3'
     3'-TCGAGG CCA CCA TCT GTT AAC TTG AGA CCA CTT AGG AC-5'

                                                              BglII

4    5'-G ACC ATC AAC GTC GAA CCA GGT AAC AAC GGT GGT AA-3'
     3'-C TGG TAG TTG CAG CTT GGT CCA TTG TTG CCA CCA TTC TAG-5'

         BglII

5    5'-G ATC TGG GCT AGA ACC GAC TGT TAC TTC GAT
         3'-ACC CGA TCT TGG CTG ACA ATG AAG CTA

                       GAC TCT GGT TCC GGT ATC TGT AAG ACT GG-3'
                       CTG AGA CCA AGG CCA TAG ACA TTC TGA CC-5'


6    5'-T GAC TGT GGT GGT TTG TTG AGA TGT AAG AGA TTC
     3'-A CTG ACA CCA CCA AAC AAC TCT ACA TTC TCT AAG        EcoRI
                       GGT AGA CCA CCA ACC ACT TTG GCT G-3'
                       CCA TCT GGT GGT TGG TGA AAC CGA CTT AA-5'

EcoRI

7  5'-AA TTC TCT TTG AAC CAA TAC GGT AAG GAC TAC ATC GAT ATC TCC AAC ATC AAG-3'
   3'-G AGA AAC TTG GTT ATG CCA TTC CTG ATG TAG CTA TAG AGG TTG TAG TTC-5'

                                                                    SstI

8  5'-GGT TTC AAC GTT CCA ATG AAC TTC TCT CCA ACC ACT AGA GGT TGT AGA GGAGCT-3'
   3'-CCA AAG TTG CAA GGT TAC TTG AAG AGA GGT TGG GCA TCT CCA ACA TCT CC-5'

   SstI                                                         HindIII

9    5'-C GTC AGA TGT GCT GCT GAC ATC GTT GGT CAA TGT CCA GCT A-3'
   3'-TCGAG CAG TCT ACA CGA CGA CTG TAG CAA CCA GTT ACA GGT CGA TTC GA-5'

   HindIII

10 5'-AG CTT AAG GCT CCA GGT GGT GGT TGT AAC GAC GCT TGT ACC GTT TTC CAA ACT-3'
      3'-A TTC CGA GGT CCA CCA CCA ACA TTG CTG CGA ACA TGG CAA AAG GTT TGA-5'

                                                                    XbaI

11 5'-TCC GAA TAC TGT TGT ACC ACT GGT AAG TGT GGT CCA ACC GAA TAC T-3'
   3'-AGG CTT ATG ACA ACA TGG TGA CCA TTC ACA CCA GGT TGG CTT ATC AGA T-5'

   XbaI                                            SstI

12 5'-CT AGA TTC TTC AAG AGA TTG TGT CCA GAC GAGCT-3'
    3'-CT AAG AAG TTC TCT AAC ACA GGT CTG C-5'

   SstI

13   5'-CT TTC TCC TAC GTC TTG GAC AAG CCA ACT ACC GTC ACT TGT CCA-3'
   3'-TCGAGA AAG AGG ATG CAG AAC CTG TTC GGT TGA TGG CAG TGA ACA GGT-5'

                                                                    XhoI

14 5'-GGT TCT TCC AAC TAC AGA GTT ACC TTC TGT CCA ACT GCC TAA TCA C-3'
   3'-CCA AGA AGG TTG ATG TCT CAA TGG AAG ACA GGT TGA CGG ATT ACT GAG CT-5'

- 15 -

The following example is directed to the preparation of three intermediate vectors, each containing a sub-assembly portion of a completely assembled manufactured gene for thaumatin I.

## EXAMPLE 3

Three portions of the synthetic thaumatin gene were constructed concurrently in three different pBR322 derived vectors. Portion A consisted of extended duplexes 1 and 2, 3 and 4, and 5 and 6; Portion B consisted of extended duplexes 7 and 8, 9 and 10 and 11; and Portion C consisted of duplexes 12 and 13 and 14. Plasmid pING233 was designed to receive Portion A, plasmid pING235 to receive Portion B, and plasmid pING237 to receive Portion C. As shown in Table V below, during construction of these plasmids new restriction enzyme sites were introduced into plasmid vector pBR322 by blunt end ligation of synthetic undecamer (11-mer) linkers and one commercially available 8-mer linker into existing restriction sites [Maniatis, et al., Cell, 15, 687-701 (1978)].

## TABLE V

| New Plasmid | Former pBR322 Resriction Endonuclease Enzyme Recognition Site | New Restriction Endonuclease Enzyme Recognition Site | Synthetic Linker |
|---|---|---|---|
| pING233 | BamHI | BglII | 5'-GGAGATCTCCC-3' |
|  | SalI | SstI | 5'-GGCGAGCTCCCG-3' |
|  | PvuII | BclI | 5'-TGATCACGCCG-3' |
| pING235 | ClaI | SstI | 5'-GGCGAGCTCCCG-3' |
|  | BamHI | XbaI | 5'-GGTCTAGAGCC-3' |
| pING237 | BamHI | XbaI | 5'-GGTCTAGAGCC-3' |
|  | SalI | SstI | 5'-GGCGAGCTCCCG-3' |
|  | PvuII | XhoI | 5'-CCTCGAGG-3' (Collaborative Research, Inc., Waltham, MA) |

Duplexes prepared as described in Examples 1 and 2 were ligated into the appropriate vector to produce a segment. The two segments formed by only one duplex, i.e., duplex 9 and duplex 12, required double digestion of the duplex and ligation to the cohesive termini of the vector. The six segments formed by ligation of two duplexes, i.e., formed by duplexes 1 and 2, 3 and 4, 5 and 6, 7 and 8, 10 and 11; 13 and 14, were created by sticky-end ligation of each cleaved duplex to the sticky ended vector and blunt end ligation to each other.

Plasmid pING233 (0.4 pmoles) was digested with restriction endonuclease enzymes BglII and SstI and dephosphorylated with calf intestinal alkaline phosphatase. The segment formed by purified duplexes 3 and 4 (1 picomole each) were digested with the same enzymes and mixed with the vector. Ligation was carried out at 12°C overnight with 400 units of T4 DNA ligase. This ligation mixture was used to transform E.coli HB101 cells. In a similar manner, the segments formed by duplexes 5 and 6 and 1 and 2, respectively, were sequentially inserted into the cloning vector containing duplexes 3 and 4, so that the resulting vector, pING249, contained Portion A of the synthetic thaumatin gene.

Plasmid pING235 was manipulated in a similar manner to insert the segments formed by duplexes 7 and 8, and 10 and 11 of Portion B of the gene. However, to clone duplex 9, further modifications in the usual protocol were required. Polymerized duplex 9 could not be digested with HindIII and thus was ligated as a duplex with one sticky and one blunt end after SstI digestion. The plasmid pING235 was digested with HindIII, filled in with DNA polymerase (Klenow) and digested with SstI to provide compatible ends for ligation of duplex 9. Thereafter the duplexes 10 and 11 and 7 and 8, respectively, were inserted to complete Portion B and form the resulting vector pING268.

Plasmid pING237, into which Portion C of the gene was to be inserted, also required modification of the usual protocol to insert the segment formed by duplexes 13 and 14. Following digestion of duplex 12 and pING237 with XbaI and SstI and ligation of duplex 12 to the cohesive termini of the vector, attempts were made to insert the segment formed by duplexes 13 and 14. Initial attempts to clone the duplexes 13 and 14 which had been cleaved with SstI and XhoI, respectively, yielded a high frequency of recombinants, all of which contained only duplex 13, sticky-end ligated at the SstI site and blunt-end ligated near the XhoI site of the vector. By inserting duplex 14 as a doubly-phosphorylated, blunt-end duplex, no XhoI nuclease-sensitive overhang was present. The vector was digested with SstI/PvuII and dephosphorylated; duplex 13 was not phosphorylated. Therefore, the vector could not reclose without the phosphorylated duplex 14. The vector containing all of the Portion C was named pING277.

The following description is directed to the screening procedures employed to ensure that proper ligation events occurred in assembly of plasmids pING256, pING270 and pING285.

To detect inversions and deletions at the point of blunt-ended ligation, a "junction-region" probe was employed for use in colony hybridization. Because the percentage destabilization produced by one base mismatch is very great and even single base deletions at the junction can be discerned very easily with a short probe [Wallace, et al., Nucleic Acids Research, 6, 3543-3557 (1979)], an undecamer was designed for each set which spanned the junction and was therefore complementary to the end of each contributor to the blunt-ended ligation.
A probe of this length hybridizes very strongly to the
correctly ligated product.

Colony hybridization was performed according
to the procedures of Grunstein, et al., PNAS-USA, 72,
3961-3965 (1975) and Wallace, et al., Nucleic Acids
Research, 9, 879-894 (1981) with the following modifications. Filters were prehybridized with 10x Denhardt's,
4x or 1x SSC, 0.5% Triton X-100 and 100 µg/ml herring
sperm DNA at 60°C for two hours. Synthetic junction
probes were radioactively labeled with [γ-$^{32}$P]ATP as
described in Sgaramella, supra. Undecamers were separated from unincorporated label by polyacrylamide gel
electrophoresis. Gene fragments (25- to 39-mers) were
centrifuged through a P-10 column to remove unreacted
ATP. Hybridization with undecamer probes was performed
at 22°C in 4x SSC for 2 to 12 hours. The filters were
subsequently washed for ten minutes in 4x SSC at the
same temperature three times. Gene fragments were
hybridized at 50°C in 1x SSC and washed in 1x SSC at
40°C, for the same intervals as the undecamers.

The following description is directed to
sequencing of the vectors.

Because the cloning of synthetic segments into
each vector was a sequential process, the newly-inserted
duplexes were sequenced to avoid error accumulation
before integration of further duplexes into the same
vector. Dideoxy chain termination sequencing was performed directly from linearized plasmid DNA as described
in Sanger, et al., PNAS-USA, 74, 5463-67 (1977) and
Wallace, et al., Nucleic Acids Research, 19, 879-894
(1981), using synthetic oligomers complementary to portions of pBR322 or gene fragments as primers. Plasmid
DNA for sequencing was prepared by a mini-lysate protocol
[Holmes, et al., Anal.Biochem., 114, 193-197 (1981)] and

rapidly purified on an RPC5 analog mini-column to remove small oligonucleotide primers [Thompson, et al., Methods in Enzymology, 100, 368-399 (1983)]. With pBR322 oligonucleotides (13-mers) and thaumatin oligonucleotides (25- to 39-mers) as primers, the reactions were performed at 30°C and 37°C, respectively.

Sequence analysis of hybridization-positive colonies determined that greater than 60% of these clones contained a deletion, insertion or base change. Single nucleotide changes were most frequent and (in 90% of the cases) consisted of G to A transitions on the synthesized strand, possibly caused by dimer impurity or incomplete deblocking of G residues before annealing and polymerization.

The following example is directed to improved methods of primer directed mutagenesis employing a linearized single-stranded plasmid template to correct the several single base changes noted above.

EXAMPLE 4

An improved process for selectively altering the nucleotide sequence of a double-stranded plasmid DNA sequence having at least two unique restriction endonuclease enzyme recognition sites was developed during the course of the construction of the thaumatin genes, and involves annealing a single-stranded primer oligonucleotide containing a selected alteration to one strand of the double-stranded DNA sequence and extending the primer to form a partial complement thereof. The improvement in this mutagenesis process comprises the steps of:

(a) linearizing the double-stranded plasmid DNA sequence by restriction endonuclease enzyme digestion at a first unique recognition site in the sequence;

(b) denaturing the linearized double-stranded DNA sequence formed in step (a) into two complementary linear single-stranded DNA sequences;

(c) annealing the primer to one of the linear single-stranded sequences formed in step (b) and extending the primer to form a partially double-stranded DNA sequence;

(d) denaturing this partially double-stranded sequence formed in step (c) into the original plasmid-derived single-stranded sequence and a primer-derived single-stranded DNA sequence;

(e) linearizing the double-stranded plasmid DNA sequence by restriction endonuclease enzyme digestion at a second unique recognition site in the sequence;

(f) denaturing the linear sequence formed in step (e) into two complementary single-stranded DNA sequences;

(g) annealing the primer-derived single-stranded DNA sequence formed in step (d) to a plasmid-derived complementary single-stranded sequence formed in step (f);

(h) recircularizing the annealed strands of step (g) into a double-stranded DNA plasmid with an alteration in one strand;

(i) transforming a host microorganism with the plasmid formed in step (h) and isolating daughter cell populations containing plasmids with the selectively altered sequence by hybridization with the primer; and

(j) transforming a host microorganism with the selectively altered plasmids obtained from the hybridization of step (i).

As one example, this complementary strand mutagenesis was employed to cause an A to C transversion in the codon specifying threonine at position number 154 of the thaumatin I sequence (see Table VI, infra). The plasmid manipulated contains duplexes 9 to 11 between the SstI and XbaI sites of pBR322 derivative pING235.

After digestion of the super-coiled plasmid with a restriction endonuclease enzyme, <u>Pst</u>I, one microgram of the now-linearized plasmid was mixed with a twenty-fold molar excess of a phosphorylated mutagenic primer having the correct sequence, 5'-CAAA$^{C}$TTCCGA-3'. In 25 μl polymerization buffer the linearized double-stranded plasmid was denatured and the primer reannealed as follows. The mixture was sealed in a capillary tube, incubated at 100°C for three minutes and plunged into an ice water bath for several minutes. Prior to polymerization the mixture was pre-incubated at the reaction temperature, 12°C, for ten minutes.

Primer elongation was carried out in 0.5 mM deoxynucleoside triphosphates, 5 mM DTT and 1 unit DNA Polymerase I, Klenow Fragment at 12°C for thirty minutes followed by 37°C for two hours. The mixture was then heated at 100°C for one minute to denature the polymerase and mixed with pING235, linearized by digestion with 1 μg <u>Ava</u>I and dephosphorylated. The reaction mixture was diluted to 100 μl, sealed in a capillary tube, heated to 100°C for three minutes and incubated at 60°C for two hours to reanneal the extended primer to a complementary strand. The desired hybrid contains different locations for the restriction endonuclease enzyme recognition site at which each strand was cleaved; thus, when completely annealed, a circular structure is formed. The mixture was desalted by centrifugation through P10, lyophilized and dissolved in 20 μl polymerase buffer. A complete, circular duplex was created by incubation with 400 units T4 DNA ligase, 0.5 mM deoxynucleoside triphosphates, 0.5 mM ATP and 1 unit DNA Polymerase I, Klenow Fragment overnight at 12°C. After transformation of <u>E.coli</u> HB101, colonies were screened by hybridization using the primer as probe.

An alternative improved process was developed for selectively altering the nucleotide sequence of a double-stranded plasmid DNA sequence having first, second and third unique restriction endonuclease enzyme recognition sites, wherein a single-stranded primer oligonucleotide containing the selected alteration is annealed to one strand of the double-stranded DNA sequence and extended to form a partial complement thereof, and the plasmid DNA sequence to be altered is between the second and third recognition sites. The improvement comprises the steps of:

(a) linearizing the double-stranded plasmid DNA sequence by restriction endonuclease enzyme digestion at the first recognition site in the sequence;

(b) denaturing the linearized double-stranded DNA sequence formed in step (a) into two complementary linear single-stranded DNA sequences;

(c) annealing the primer to one of the linear single-stranded sequences formed in step (b) and annealing to the same linear sequence a second primer complementary to a portion of the sequence between the first and second recognition sites and extending the primers to form a partially double-stranded DNA sequence including both the second and third recognition sites;

(d) cleaving the partially double-stranded sequence formed in step (c) by restriction endonuclease digestion at the second and third restriction sites to form a double-stranded sequence which is a hybrid of a plasmid single strand and the primer extended strands;

(e) inserting the fragment formed in step (d) into the DNA plasmid to form a double-stranded plasmid including the alteration in one of its strands;

(f) transforming a host microorganism with the plasmid formed in step (e) and isolating daughter cell populations containing double-stranded plasmids with the selectively altered sequence by hybridization with the primer; and

(g) transforming a host microorganism with the selectively altered plasmids obtained from the hybridization of step (f).

As one example, this fragment excision/religation mutagenesis technique was employed to correct a G to A transition in the center of the codon specifying glycine at position number 144 of the thaumatin I gene. The second primer was employed upstream from the mutagenic primer, which, when elongated, reformed the recognition site. One microgram of super-coiled plasmid containing duplexes 7-11 inserted between the EcoRI and XbaI sites of pING235 was digested to completion with restriction endonuclease enzyme PstI. This plasmid was combined with 30 ng (6 pmole) each: (a) phosphorylated mutagenic primer containing the alteration, 5'-CAACCA$^C$CACC-3'; and (b) a pBR322 primer, 5'-GTTGAAGGCTCTC-3', which is complementary to the sequence adjacent to the SalI site and primes counterclockwise. The mixture was denatured and the primers annealed as described above for the complementary strand mutagenesis. Prior to polymerization and ligation the solution was pre-incubated at the reaction temperature (12°C) for five minutes and then the solution was adjusted as follows: 0.5 mM in each dNTP, 5 mM DTT, 1 unit DNA Polymerase I, Klenow Fragment, 0.5 mM in ATP, and 400 units T4 DNA ligase. The mixture was incubated at 12°C overnight, the enzymes denatured and the restriction enzymes XbaI and EcoRI used to excise a portion of the newly-copied region. This was mixed with the equivalently digested, dephosphorylated pING235-derivative plasmid containing duplexes 7-11 and ligated at 12°C overnight. The mixture was used to transform HB101 and ampicillin-resistant colonies were screened by colony hybridization using the primer containing the alteration as the probe.

The following example is directed to the final assembly of the gene from the corrected portions A, B and C of plasmids pING249, pING268 and pING277.

## EXAMPLE 5

After correction of all the nucleotide errors by _in vitro_ mutagenesis in Example 4, the SstI site (5'-GAGCTC-3') of each completed gene portion was destroyed by removal of the AGCT sequence. Each plasmid was digested to completion with SstI and adjusted to 0.5 mM in each deoxynucleotide triphosphate. Removal of the 3' single-stranded end was accomplished by the addition of 1 unit of DNA Polymerase I (Klenow) and incubation of the mixture at 22°C for 15 minutes. T4 DNA ligase was then added (400 units) and the solution placed at 12°C overnight. The ligation mixture was used to transform HB101 and ampicillin-resistant colonies were screened for the SstI site. Following these procedures, the resulting plasmids were re-designated. pING249 became pING250; pING268 became pING270; and pING277 became pING278.

pING250 was thereafter digested with BclI enzyme and blunt-ended by S1 nuclease. A BamHI linker of sequence 5'-CGGGATCCCG-3' (New England Biolabs) was inserted between the blunt ends to generate pING256. An additional "stop" codon was introduced into pING278 by primer-directed mutagenesis to generate pING285.

The three completed portions of the gene present in plasmids pING256, pING270 and pING285 were then combined into one complete gene sequence, as set out in Table VI, below.

## TABLE VI

BclI

                                      HpaI

```
        -1   1                                        10
        Met Ala Thr Phe Glu Ile Val Asn Arg Cys Ser Tyr Thr Val Trp Ala Ala Ala Ser
  ─────────────────1a─────────────────                        ───────────────────────2a
  GATC ATG GCT ACC TTC GAA ATC GTT AAC AGA TGT TCT TAC ACT GTT TGG GCT GCT GCT TCC
       TAC CGA TGG AAG CTT TAG CAA TTG TCT ACA AGA ATG TGA CAA ACC CGA CGA CGA AGG
                        ────────────────────1b──────────────────

        20                                        30
        Lys Gly Asp Ala Ala Leu Asp Ala Gly Gly Arg Gln Leu Asn Ser Gly Glu Ser Trp Thr
  ──────────────────────              ────────────3a──────────────                   ───────
  AAG GGT GAC GCT GCT TTG GAC GCC GGT GGT AGA CAA TTG AAC TCT GGT GAA TCC TGG ACC
  TTC CCA CTG CGA CGA AAC CTG CGG CCA CCA TCT GTT AAC TTG AGA CCA CTT AGG ACC TGG
       ──────────────2b──────────────              ──────────────3b──────────────

                        KpnI                        BglII
        40                                        50
        Ile Asn Val Glu Pro Gly Thr Asn Gly Gly Lys Ile Trp Ala Arg Thr Asp Cys Tyr
  ───────────4a──────────────              ────────────────────────5a──────────
  ATC AAC GTC GAA CCA GGT AAC AAC GGT GGT AAG ATC TGG GCT AGA ACC GAC TGT TAC
  TAG TTG CAG CTT GGT CCA TTG TTG CCA CCA TTC TAG ACC CGA TCT TGG CTG ACA ATG
                        ──────────────4b──────────────                          ───

        60                                        70
        Phe Asp Asp Ser Gly Ser Gly Ile Cys Lys Thr Gly Asp Cys Gly Gly Leu Leu Arg
                                            ────────────────────────────────6a───
  TTC GAT GAC TCT GGT TCC GGT ATC TGT AAG ACT GGT GAC TGT GGT GGT TTG TTG AGA
  AAG CTA CTG AGA CCA AGG CCA TAG ACA TTC TGA CCA CTG ACA CCA CCA AAC AAC TCT
  ────────────────────────5b──────────────
```

# TABLE VI (cont'd.)

```
                                                            EcoRI
              80                                            90
Cys Lys Arg  Phe Gly Arg Pro Pro Thr Thr Leu Ala Glu Phe Ser Leu Asn Gln Tyr
_____                           _____7a_____
TGT AAG AGA  TTC GGT AGA CCA CCA ACC ACT TTG GCT GAA TTC TCT TTG AAC CAA TAC
ACA TTC TCT  AAG CCA TCT GGT GGT TGG TGA AAC CGA CTT AAG AGA AAC TTG GTT ATG
            _____6b_____


                   EcoRIV
              100
Gly Lys Asp  Tyr Ile Asp Ile Ser Asn Ile Lys Gly Phe Asn Val Pro Met Asn Phe
_____                           _____8a_____
GGT AAG GAC  TAC ATC GAT ATC TCC AAC ATC AAG GGT TTC AAC GTT CCA ATG AAC TTC
CCA TTC CTG  ATG TAG CTA TAG AGG TTG TAG TTC CCA AAG TTG CAA GGT TAC TTG AAG
            _____7b_____


              120                                          130
Ser Pro Thr  Thr Arg Gly Cys Arg Gly Val Arg Cys Ala Ala Asp Ile Val Gly Gln
_____                           _____9a_____
TCT CCA ACC  ACT AGA GGT TGT AGA GGC GTC AGA TGT GCT GCT GAC ATC GTT GGT CAA
AGA GGT TGG  GCA TCT CCA ACA TCT CCG CAG TCT ACA CGA CGA CTG TAG CAA CCA GTT
            _____8b_____


              HindIII 140                                  150
Cys Pro Ala  Lys Leu Lys Ala Pro Gly Gly Gly Cys Asn Asp Ala Cys Thr Val
_____10a_____
TGT CCA GCT  AAG CTT AAG GCT CCA GGT GGT GGT TGT AAC GAC GCT TGT ACC GTT
ACA GGT CGA  TTC GAA TTC CGA GGT CCA CCA CCA ACA TTG CTG CGA ACA TGG CAA
9b_____               _____10b_____
```

TABLE VI (cont'd.)

```
                                                                                    XbaI
                                          160                                       170
Phe Gln Thr Ser Glu Tyr Cys Cys Thr Thr Gly Lys Cys Gly Pro Thr Glu Tyr Ser Arg
————————————————————————————————11a————————————————————
TTC CAA ACT TCC GAA TAC TGT TGT ACC ACT GGT AAG TGT GGT CCA ACC GAA TAC TCT AGA
AAG GTT TGA AGG CTT ATG ACA ACA TGG TGA CCA TTC ACA CCA GGT TGG CTT ATC AGA TCT
————————————————————                    ——————————————————————11b——————————

                                          180                                       190
Phe Phe Lys Arg Leu Cys Pro Asp Ala Phe Ser Tyr Val Leu Asp Lys Pro Thr Thr Val
——————————12a——————————             ——————————————————13a——————————
TTC TTC AAG AGA TTG TGT CCA GAC GCT TTC TCC TAC GTC TTG GAC AAG CCA ACT ACC GTC
AAG AAG TTC TCT AAC ACA GGT CTG CGA AAG AGG ATG CAG AAC CTG TTC GGT TGA TGG CAG
          ——————————————12b——————————     ——————————————————13b——————————

                                          200                     207
Thr Cys Pro Gly Ser Ser Asn Tyr Arg Val Thr Phe Cys Pro Thr Ala
————————————————————————————14a——————————————————
                                                                   XhoI
ACT TGT CCA GGT TCT TCC AAC TAC AGA GTT ACC TTC TGT CCA ACT GCC TAA TCA C
TGA ACA GGT CCA AGA AGG TTG ATG TCT CAA TGG AAG ACA GGT TGA CGG ATT ACT GAG CT
————————————————————             ——————————————————————14b——————————
```

Simultaneous integration of two gene portions into the vector containing the third, pING285, was accomplished by taking advantage of the requirement for an intact ampicillin-resistant gene in viable colonies and destroying the integrity of this gene on the two plasmids donating remaining gene portions. Thereafter, plasmids pING256, containing duplexes 1-6, and pING270, containing duplexes 7-11, were linearized with PstI and the resultant 3' single-stranded regions were degraded by the action of DNA polymerase I (Klenow). After ligation, the ampicillin-resistant gene contained a frameshift which prevented expression of an active gene product. The gene portions were subsequently excised with BamHI and EcoRI for pING256 and EcoRI and XbaI for pING270. The vector which accepted the two restriction fragments (pING285, which contained oligonucleotide duplexes 12-14) was alkaline phosphatase treated to prevent closure without the phosphorylated restriction fragments from pING256 and pING270. The resulting plasmid, containing the entire synthetic thaumatin I gene, was named pING301. 50% of the colonies isolated contained the expected restriction map of the thaumatin gene. Sequence analysis demonstrated that the entire coding sequence was present.

The following example is directed to the construction of a fused gene comprising the entire thaumatin coding sequence of pING301 inserted in the proper reading frame immediately following the first 426 nucleotides of the S.typhimurium araB gene.

## EXAMPLE 6

In this construction, plasmid pMH6, a derivative of pMH3 [Horwitz, et al., Gene, 14, 309-319 (1981)], was employed. Plasmid pMH6 harbored in E.coli strain MC1061 has been deposited with the American Type Culture Collection, 12301 Parklawn Drive, Rockville, Maryland 20852, under the identifying number ATCC 39450. pMH6 was digested with SalI and EcoRI and the M13mp9 poly-linker fragment [Messing, J., in Third Cleveland Symposium on Macromolecules: Recombinant DNA, (Walton, A., ed.) Elsevier, Amsterdam 143-153 (1981)] was inserted into the digested plasmid. In order to direct the integration of the thaumatin I gene, an XhoI linker was ligated into the T4 DNA polymerase filled-in EcoRI site. This resulting vector, pING61, was digested at the SalI site of the araB gene, and the single-stranded region was filled in with DNA polymerase I (Klenow), thereby blunt-ending that site. After subsequent XhoI digestion, this vector was ligated with the thaumatin gene derived from pING301 (BamHI-digested at the 5' end of the thaumatin I gene, filled in, and XhoI-digested) to form plasmid pING307. Competent E.coli strain MC1061 [Casadaban, et al., J.Mol.Biol., 138, 179-207 (1980)] cells were used for transformation. Clones containing the araB gene of pMH6 fused to the entire thaumatin I gene were obtained and confirmed for precise joining by sequence analysis of the fusion junction.

The following example is directed to an alternative vector for obtaining E.coli expression of the thaumatin I gene constructed in the foregoing examples.


EXAMPLE 7

The commercially available tac promoter [Russell, et al., Gene, 20, 231-243 (1982)] was used to achieve expression of the thaumatin gene in E.coli. The vector used, pDR540, [PL Biochemicals, Milwaukee, Wis.] contains a BamHI site directly after the Shine-Dalgarno sequence of the tac promoter, facilitating insertion and expression of a synthetic gene without a ribosomal binding site. Downstream from this site is a galactose kinase gene, also under control of the tac promoter. The XhoI site at the 3' end of the thaumatin gene was converted to BamHI by digestion of pING301 with XhoI, filling in with DNA polymerase (Klenow) and blunt-end ligation with an XhoI linker (Collaborative Research, Inc.). The thaumatin gene was removed from the resulting vector (pING302) by digestion with BamHI, purified from the remainder of the plasmid by agarose gel electrophoresis and ligated with BamHI-digested pDR540 at 12°C overnight with 400 units T4 DNA ligase to yield plasmid pING304. This plasmid was used to transform E.coli strain 71.18, an E.coli JM101 (ATCC 33876) derivative with a change in one gene locus. E.coli strain 71.18 may be produced by employing the standard procedure of Miller, J.H., Experiments in Molecular Genetics, Cold Spring Harbor Laboratory, Cold Spring Harbor, New York (1972). Briefly put, a culture of E.coli cells (ATCC 33760) which carries the tra gene is infected with bacteriophage P1 (ATCC e25404-B1). The bacteriophage then lyses the cells and packages the chromosomal tra gene DNA. JM101 cells are thereafter transduced with the tra gene-carrying phage, resulting in E.coli 71.18. Isopropyl β-D thiogalactoside (IPTG) induces the tac promoter, which is normally repressed in strain 71.18. Ampicillin-resistant colonies were screened by colony hybridization with the thaumatin gene oligonucleotide 5b (see Table II) as a probe. Positive colonies were screened for the proper thaumatin orientation by mapping with EcoRI digestion.

The following example is directed to determination of the stability of the thaumatin I polypeptide produced in the expression systems of Examples 6 and 7.

## EXAMPLE 8

Cultures were grown in minimal medium containing glycerol (0.2%) as a carbon source, thiamine (2 µg/ml), and ampicillin (100 µg/ml) for strain 71.18 containing pING304 and with the addition of leucine (0.004%) for strain MC1061 containing pING307.

To perform pulse-labeling experiments with [$^{35}$S]cysteine, cells were grown to an $A_{600\ nm}$ of 0.1 and induced with 1% arabinose (MC1061) or 1 mM isopropyl β-D thiogalactoside (71.18). After a thirty-minute incubation, 10 µCi [$^{35}$S]cysteine or [$^{35}$S]methionine (10 pmoles) was added. The cells were chilled on ice after the 5-minute desired labeling period, pelleted by centrifugation for one minute in a microfuge, and lysed by boiling for five minutes in 20 mM Tris-Cl, pH 7.2, 1% SDS. Pulse-chase experiments with [$^{35}$S]cysteine to determine the stability of synthesized thaumatin in vivo were performed equivalently, except that unlabeled cysteine (50 µg/ml) was added after a five-minute labeling period. Cells were harvested at varying times thereafter.

For MC1061 cells transformed with plasmid pING307 including the araB-thaumatin fusion gene of Example 6, autoradiography of SDS-polyacrylamide gel analysis of [$^{35}$S]cysteine-labeled extracts demonstrated the presence of a fusion protein of the expected molecular weight, 36 kilodaltons. This protein was not visible in the uninduced extracts. Neither a radioimmunoassay nor a gel stained with Coomassie Blue were sufficiently sensitive to reveal the presence of this fusion polypeptide.

In 71.18 cells transformed with plasmid pING304 having the thaumatin gene under tac control, during a five-minute pulse labeling with [$^{35}$S]cysteine a protein the size of thaumatin was produced. After a five-minute chase greater than 90% of the thaumatin had been degraded, indicating that this protein is unstable in this bacterial strain. Neither uninduced nor [$^{35}$S]methionine-labeled extracts produce this protein at detectable levels. This result is consistent with the frequency of occurrence of these two amino acids in thaumatin (16 times vs. 1 time). As in the fusion protein case, neither a radioimmunoassay nor a Coomassie Blue stained gel detected the presence of this protein.

The following example is directed to manipulations performed to confirm the identity of the fusion and thaumatin polypeptides, produced in the previous examples.

### EXAMPLE 9

Anti-thaumatin antibodies were raised by multiple subcutaneous injections of adult male New Zealand rabbits with 400 μg thaumatin in Freund's Complete Adjuvant. Booster injections were with 80 μg thaumatin. The IgG fraction was isolated by ammonium sulfate precipitation [Weickman, J.L., et al., Biochem., 20, 1272-1278 (1981)].

SDS-lysed, radiolabeled extracts of induced cells were immunoprecipitated with the above antibody preparation as described by Sen, et al., [PNAS-USA, 80, 1246-1250 (1983)]. [$^{125}$I]iodothaumatin as a gel standard was prepared as described by Weickmann, et al., [J.Biol. Chem., 257, 8705-8710 (1982)] for iodinating proteins. [$^{35}$S]cysteine-labelled cell extracts were immunoprecipitated, electrophoresed through a polyacrylamide gel and autoradiographed. Extracts from pING304 transformed 71.18 cells (Example 7) showed one protein band, identical in mobility to a thaumatin standard. In contrast, extracts of pING307 transformed MC1061 cells of Example 6 showed both a protein of the predicted size and a band of slightly higher mobility. This second band occurred only in induced extracts with immune, but not pre-immune serum. Pulse-chase experiments demonstrated that the initial five-minute labeling produced only the larger of these two proteins. During the subsequent chase period the higher mobility band appeared and became equal in intensity to the original band after twenty minutes. This lower molecular weight, immunoprecipitable protein may be a specific degradation product of the fusion polypeptide.

The following example is directed to vector constructions for expression of the thaumatin gene in yeast cells.

## EXAMPLE 10

### A.  The Construction of pING60

To express the thaumatin gene in yeast, the PGK gene from Saccharomyces cerevisaie was isolated from a yeast genomic bank using colony hybridization (as described in Example 3) with a 17-mer synthetic probe complementary to the published PGK promoter sequence [Dobson, et al., Nucleic Acid Res., 10, 2625-2637 (1982)]. A 3kb HindIII fragment containing the PGK gene was subcloned into E.coli pBR322 to generate hybrid plasmid pPGK-p. pPGK-p was thereafter digested with MboII to obtain a 218 bp MboII fragment containing the proximal end of the PGK promoter from pPGK-p. This fragment was reacted with T4 DNA polymerase to produce blunt ends and ligated to BclI-digested and blunt-ended pING250 (Example 5). The resulting plasmid, pING51, contained an MboII site re-created at only the 5' end of the PGK promoter. Plasmid pING51 was redigested with MboII, and treated with T4 DNA polymerase to produce blunt ends. After attachment of BamHI linkers at the blunt-ended MboII site, pING51 was digested with EcoRI to remove the PGK-thaumatin (portion A) sequence, which was thereafter joined to BamHI and EcoRI digested

pING301. The complete thaumatin sequence was contained in the resulting plasmid, pING52.

Plasmid pING52 was digested with <u>Bam</u>HI, filled in with T4 DNA polymerase, and then digested with <u>Eco</u>RI to generate a PGK promoter-thaumatin (portion A) fragment which contained a blunt end at the 5' terminus and a <u>Eco</u>RI sticky end at the 3' terminus. pPGK-p was digested with <u>Xba</u>I, treated with T4 DNA polymerase to fill in the ends, and digested with <u>Eco</u>RI, thereby removing a portion of the plasmid's intact PGK structural gene. The insertion of the PGK promoter-thaumatin (portion A) fragment into the digested pPGK-p formed pING55a, which thereby contained tandem PGK promoters. A deletion was generated between the duplicated promoter regions through <u>in vivo</u> recombination in <u>E.coli</u>, placing the intact PGK promoter immediately upstream from the 5' end of the thaumatin gene. A representative clone, designated pING56a, contained only portion A of the thaumatin gene. The PGK-thaumatin (portion A) fragment was removed from pING56a by a <u>Bam</u>HI/<u>Eco</u>RI double digestion and joined to the same restriction sites in pING52, resulting in plasmid pING57 which contained the complete PGK promoter and transcriptionally active thaumatin gene on a <u>Bam</u>HI/<u>Xho</u>I fragment.

To create an <u>E.coli</u>-yeast shuttle vector to receive this <u>Bam</u>HI/<u>Xho</u>I fragment, plasmid pPGK-p was digested with <u>Bgl</u>II and <u>Eco</u>RI and treated with T4 DNA polymerase to produce blunt ends. <u>Xho</u>I linkers were attached, and the plasmid was religated and transformed into <u>E.coli</u> strain MC1061 to generate pING53, in which the PGK terminator was located between the <u>Xho</u>I and <u>Hind</u>III sites. A <u>Bam</u>HI/<u>Hind</u>III fragment containing the PGK terminator and 190 base pairs of pBR322 DNA was then joined to the yeast-<u>E.coli</u> shuttle vector, pJDB209 [Beggs, J., "Multicopy Yeast Plasmid Vectors" in <u>Molecular Genetics in Yeast</u>, von Wettstein, <u>et al.</u>, eds. (Copenhagen 1981)], at the <u>Bam</u>HI and <u>Hind</u>III sites to

create pING58. Plasmid pJDB209 harbored in host cell E.coli K12 strain MC1061 has been deposited with the American Type Culture Collection, 12301 Parklawn Drive, Rockville, Maryland 20852 and designated ATCC 39449. The BamHI/XhoI fragment from pING57 was then ligated to the same restriction sites in pING58, resulting in the PGK terminator being positioned downstream from the thaumatin gene.

The resultant chimeric plasmid pING60 containing the thaumatin gene was inserted between the yeast phosphoglycerate kinase gene (PGK) promoter and terminator. Yeast strain AH-22 [Ito, et al., J.Bacteriology, 153, 163-168 (1983)] (ATCC 38626) was used as a recipient for expression of the synthetic thaumatin gene in pING60.

Yeast strains carrying the plasmid pING60 were cultured in SD(-)leu medium, a synthetic complete medium consisting of nitrogen base without amino acids, 2% glucose, amino acid supplements without leucine, and purine and pyrimidine supplements, since the PGK promoter in this plasmid is constitutive when cells are grown in the presence of glucose as the sole carbon source. Single colony yeast transformants containing the plasmid pING60 were inoculated into 15 ml of SD(-) leucine broth and grown to saturation at 30°C with vigorous shaking ($A_{600nm}$ approximately 2.1). The saturated cultures were precooled on ice and washed twice with PBS before lysis.

B. The Construction of the Plasmid pING114

In the construction of plasmid pING114, two intermediate plasmids were employed for the purpose of contributing the raffinose-inducible yeast cyc1 promoter (pING103) and the cyc1 terminator (pING108). Both pING103 and pING108 have been deposited with the American Type Culture Collection, 12301 Parklawn Drive, Rockville, Maryland 20852, harbored in bacterial host strain E.coli K12 strain HB101.  These deposits are identified by ATCC No. 39447 and No. 39448, respectively.  In order to facilitate fusion of the cycl terminator to the thaumatin gene in the proper orientation, the SalI site 5' to the cycl terminator and the HindIII restriction site 3' to the terminator on pING108 were replaced with XhoI and SalI sites by linker addition, respectively, generating pING110 in which the direction of transcription proceeds from the XhoI site to the SalI site.

Plasmid pING301, containing the 650 base pair thaumatin gene bounded 5' by a BamHI site and 3' by a XhoI site, was BamHI digested and blunt-ended by treatment with DNA polymerase (Klenow).  Following digestion with XhoI, the 650 base pair blunt-end-XhoI thaumatin fragment was cloned into XhoI/PvuII-digested pING110, thereby regenerating a BamHI site at the 5' end of the thaumatin gene and resulting in fusion of the 3' end of the thaumatin gene to the cycl terminator in plasmid pING111.

Plasmid pING111 was digested with SalI and blunt-ended and then digested with BamHI.  The thaumatin-cycl terminator fusion was cloned into the high copy number yeast vector, pJDB209 between the BamHI site and the HindIII site which was converted to a blunt end, generating pING112.

To remove the undesirable first 7 base pairs of the cycl gene coding region, which occur between the 5' SalI site and the cycl promoter in pING103, pING103 was cleaved with EcoRI and a limited Bal31 exonuclease digestion was performed.  The plasmid was then digested with SalI, generating an array of cycl promoters that have a common SalI site at one end and a different Bal31 deletion end point at the other.  The cycl fragments with Bal31 deletion sites determined by gel electrophoresis ranging between 4 and 20 base pairs were isolated and cloned into pING112 digested with SalI and BamHI and blunt-ended.

The plasmid pING114 contains the synthetic thaumatin gene fused between the raffinose inducible yeast cyc1 gene promoter and terminator. Yeast cell transformation was performed as described for pING60 and cells were grown under either repressed conditions with (YEPD) basic complete medium containing 1% yeast extract, 2% bactopeptone and 2% dextrose, or derepressed (SD(-)leu) conditions. The latter involved addition of 2% raffinose as the sole carbon source. Single colony yeast transformants carrying the plasmids were grown overnight in 5 ml of YEPD medium and then washed twice with sterile water. A low density inoculum ($A_{600nm}$ approximately 0.1) was used to initiate growth in 15 ml cultures of YEPD or SD(-) leucine and raffinose medium. Cultures were harvested by centrifugation when cells attained an $A_{600nm}$ density of at least 2 and were treated as described above.

The following example is directed to preparation and fractionation of yeast cell extracts and analysis of the polypeptide produced therein.

## EXAMPLE 11

### A. Preparation of Yeast Cell Extracts

One milliliter of lysis buffer (20 mM Tris-Cl, 1% SDS, pH 7.2) was added to each 0.5 g of cells prepared as described above in Example 10 and the suspension boiled for 10 minutes to achieve cell breakage. The samples were then centrifuged at 14000 x g to clarify the supernatant and these were subsequently analyzed by electrophoresis through a 15% discontinuous SDS-polyacrylamide gel (SDS-PAGE). Gels of cell extract were shown to contain a protein comigrating with a thaumatin standard. Protein was measured by a variation of the method of Lowry, et al., J.Biol.Chem., 193, 265-275 (1951) with crystalline bovine serum albumin as a standard.

Yeast cells (1 ml) containing the thaumatin gene under control of the PGK promoter (pING60) were grown in SD(-)leu medium, to which was added 10 μCi of [$^{35}$S]cysteine. The cells were allowed to continue growth for 1.5 hours at 30°C with shaking after which they were harvested and lysed by boiling in pH 7.2 Tris buffer containing 1% SDS, as described above. Extracts (200,000 cpm per sample) and [$^{14}$C] molecular weight markers (BRL) were fractionated by SDS-PAGE. After drying the gel, autoradiography was used to detect newly synthesized protein bands which showed high levels of [$^{35}$S]cysteine incorporation. A prominent band absent from cell extracts not containing the thaumatin gene appeared at about 22K which comigrated with a sample of pure [$^{125}$I]thaumatin. The same procedures performed on yeast clones containing the thaumatin gene under control of the cycl promoter also showed synthesis of a new protein that comigrated with a [$^{125}$I] labelled thaumatin standard.

The newly synthesized protein present in yeast extracts containing the thaumatin gene was also detected by protein staining. Prominent bands were noted in the extracts of the clone containing pING60 whose migration is coincident with purified thaumatin. Visualization by Coomassie blue staining and comparison of band intensity with respect to the other yeast proteins indicates that the thaumatin I protein comprises about 10-15% of the SDS-soluble protein when PGK controls expression and 2-3% when cycl is the promoter. Yields of polypeptive product were in the range of approximately 10 mg OD/liter.

## B. Western Blot Analysis of Yeast Extracts

Yeast cell extracts fractionated by SDS-PAGE were analyzed for synthetic thaumatin by a Western Blot. A Bio Rad Trans Blot Cell was used to transfer the fractionated yeast proteins from a polyacrylamide gel to 0.45 micron nitrocellulose paper by electrophoresis at 50 mA for 14 hours. When electrophoresis was complete, the paper was immersed in 20 ml of 10 mM Tris-Cl, 0.9% NaCl, pH 7.4 (Buffer B) containing 5% BSA for 30 minutes, in 20 ml of Buffer B containing 5% BSA and 0.2 ml thaumatin-specific antiserum (TH2, 5/26/83) for 90 minutes, and then washed extensively with buffer B containing 0.05% Triton-x-100. A third incubation for 60 minutes with Buffer B containing 5% BSA and 100 µCi $^{125}$I-protein A followed by extensive washing labeled only those yeast proteins that specifically reacted with anti-thaumatin antibodies. These were detected by autoradiography. Two lanes contained 1 µg of thaumatin as a standard. Five lanes showed a new protein present in several yeast cell extracts that cross-reacted with thaumatin specific antibodies and migrated coincident with pure thaumatin. Extracts from cells not containing the thaumatin gene showed no antibody binding and thus no labeling with [$^{125}$I] protein A. The Western Blot clearly shows a protein identical in relative mobility (molecular weight) to purified thaumatin that is recognized specifically by thaumatin antibodies.

## C. Radioimmunoassay

A competitive binding radioimmunoassay (RIA) procedure based on published methods [Weickmann, J.L., et al., Biochemistry, 20, 1272-1278 (1981); and Parker, C.W., Radioimmunoassay of Biologically Active Compounds, Prentice Hall, Englewood Cliffs, New Jersey, 1976)] was used to detect and quantitate thaumatin in crude cell extracts. Iodinated RNase (usually 4-6 x $10^4$ cpm), 200 µl of 0.1 mg/ml of nonimmune rabbit serum and any competing antigen were mixed in a total volume of 400 µl of Buffer A (0.05M Tris-Cl, 0.15M NaCl, and 1 mg/ml of egg albumin, pH 7.5). Then 10 µl of an appropriate dilution (usually 2 x $10^3$) of antiserum was added. The entire mixture was incubated in capped Beckman Bio-Vials at 4°C for 8-16 hours followed by the addition of 200 µl of Buffer A containing sufficient goat anti-rabbit gamma-globulin to fully precipitate the rabbit globulins. A Beckman 5500 counter was used to measure the amount of [$^{125}$I] in each sample. The samples were then incubated at 4°C for 4 hours, followed by centrifugation at 12000 rpm (22000x g) for 15 minutes. The supernatants were removed by aspiration and radioactivity measured in the precipitates. In the absence of inhibitors, 75-80% of the total [$^{125}$I] was precipitated; background precipitation by non-immune serum alone was about 4% of the total [$^{125}$I]. Accurate quantitation of 8-50 ng of thaumatin was possible and sensitivity can easily be increased by a factor of 10. The point of 50% inhibition of binding of [$^{125}$I] thaumatin is 20ng of cross-reacting protein when 10 µl of a 1:2000 dilution of specific antiserum is used.

When cell extracts containing newly synthesized thaumatin were used as competitors in the RIA, inhibition of [$^{125}$I] labelled purified thaumatin for antibody binding sites was observed despite losses due to SDS interference in the assay and denaturation of the protein caused by boiling.

Numerous modifications and variations in the invention are expected to occur to those skilled in the art upon consideration of the foregoing description.

As one example, while the foregoing illustrative Examples are directed to the manufacture of a structural gene for microbial expression of thaumatin I, the present invention also comprehends the manufacture of genes coding for thaumatin II, which differs from thaumatin I in only five amino acid residues.

The thaumatin II coding sequence can be constructed by employing the above-noted primer-directed mutagenesis fragment excision/religation techniques to change the five amino acid residues in the thaumatin I sequence. Three of the amino acid changes ($Asn^{46}$ to $Lys^{46}$, $Ser^{63}$ to $Arg^{63}$, and $Lys^{67}$ to $Arg^{67}$) could be accomplished in a single mutagenesis procedure with two primers. The first primer would span the KpnI site and change the residue number 46 codon from AAC to AAG. The second primer would contain the base changes necessary to effect the changes at residue number 63 and at residue number 67. A minimum of two base changes is required to alter the codon specifying $Ser^{63}$ to one specifying arginine at that position (e.g., TCC to CGC) and three base changes are needed to secure alteration to the preferred codon for yeast expression, AGA. Similarly, while only a single base change is needed to alter the codon specifying $Lys^{67}$ to one specifying $Arg^{67}$ (e.g., AAG to AGG), two base changes are needed to secure alteration to the preferred codon, AGA. Providing glutamine in place of arginine at residue number 76 would require two base changes, i.e., AGA to CAA, which could be performed in a separate mutagenesis procedure. Similarly, another procedure would be employed to effect the codon change from AAC to GAC to replace asparagine with aspartic acid at residue number 113.

Alternatively, the changes in amino acid residues 46, 63, 67 and 76 could be effected by synthesis of four oligonucleotide fragments which are duplexed, polymerized and digested to replace the KpnI and EcoRI duplex of the thaumatin I sequence in pING301. The change at residue 113 would nevertheless require the in vitro mutagenesis procedure as described above.

Yet another example of expected modification of DNA sequences of the present invention involves the manufacture of genes for thaumatin analogs. One attempt to alter the sweetness intensity of the polypeptide includes selectively changing several of the lysine amino acid residues in the thaumatin I sequence to uncharged amino acids (i.e., glycine, valine). Similarly, the thaumatin I DNA sequence may be truncated at a variety of positions at either or both of its 5' and 3' termini to secure microbial expression of thaumatin polypeptide fragments primarily responsible for eliciting the sweetness response.

Consequently, only such limitations as appear in the appended claims should be placed on the invention. The features disclosed in the foregoing description and in the following claims may, both separately and in any combination thereof, be material for realising the invention in diverse forms thereof.

CLAIMS

1. A manufactured gene capable of directing the synthesis in a selected host microorganism of a polypeptide having one or more of the biochemical or immunological properties of thaumatin.

2. The gene according to claim 1 wherein said polypeptide has one or more of the biochemical or immunological properties of thaumatin I.

3. The gene according to claim 1 wherein said polypeptide has one or more of the biochemical or immunological properties of thaumatin II.

4. A manufactured gene according to claim 1 wherein the base sequence includes one or more codons, selected from among alternative codons specifying the same amino acid, on the basis of preferential expression characteristics of the codon in a projected host microorganism.

5. A manufactured gene according to claim 1 wherein the base sequence includes one or more codons, selected from among alternative codons specifying the same amino acid, on the basis of preferential expression characteristics of the codon in E.coli.

6. A manufactured gene according to claim 1 wherein the base sequence includes one or more codons, selected from among alternative codons specifying the same amino acid, on the basis of preferential expression characteristics of the codon in Saccharomyces cerevisiae.

7. A manufactured gene according to claim 1 comprising the following sequence of nucleotide bases in the coding strand thereof:

```
       1                                                    10
       Ala Thr Phe Glu Ile Val Asn Arg Cys Ser Tyr Thr Val
     5'-GCT ACC TTC GAA ATC GTT AAC AGA TGT TCT TAC ACT GTT

                               20
       Trp Ala Ala Ala Ser Lys Gly Asp Ala Ala Leu Asp Ala Gly
       TGG GCT GCT GCT TCC AAG GGT GAC GCT GCT TTG GAC GCC GGT

               30                                     40
       Gly Arg Gln Leu Asn Ser Gly Glu Ser Trp Thr Ile Asn Val
       GGT AGA CAA TTG AAC TCT GGT GAA TCC TGG ACC ATC AAC GTC

                                           50
       Glu Pro Gly Thr Asn Gly Gly Lys Ile Trp Ala Arg Thr Asp
       GAA CCA GGT ACC AAC GGT GGT AAG ATC TGG GCT AGA ACC GAC

                       60
       Cys Tyr Phe Asp Asp Ser Gly Ser Gly Ile Cys Lys Thr Gly
       TGT TAC TTC GAT GAC TCT GGT TCC GGT ATC TGT AAG ACT GGT

       70                                             80
       Asp Cys Gly Gly Leu Leu Arg Cys Lys Arg Phe Gly Arg Pro
       GAC TGT GGT GGT TTG TTG AGA TGT AAG AGA TTC GGT AGA CCA

                               90
       Pro Thr Thr Leu Ala Glu Phe Ser Leu Asn Gln Tyr Gly Lys
       CCA ACC ACT TTG GCT GAA TTC TCT TTG AAC CAA TAC GGT AAG

               100                                    110
       Asp Tyr Ile Asp Ile Ser Asn Ile Lys Gly Phe Asn Val Pro
       GAC TAC ATC GAT ATC TCC AAC ATC AAG GGT TTC AAC GTT CCA

                                       120
       Met Asn Phe Ser Pro Thr Thr Arg Gly Cys Arg Gly Val Arg
       ATG AAC TTC TCT CCA ACC ACT AGA GGT TGT AGA GGC GTC AGA

               130
       Cys Ala Ala Asp Ile Val Gly Gln Cys Pro Ala Lys Leu Lys
       TGT GCT GCT GAC ATC GTT GGT CAA TGT CCA GCT AAG CTT AAG

       140                                    150
       Ala Pro Gly Gly Gly Cys Asn Asp Ala Cys Thr Val Phe Gln
       GCT CCA GGT GGT GGT TGT AAC GAC GCT TGT ACC GTT TTC CAA

                               160
       Thr Ser Glu Tyr Cys Cys Thr Thr Gly Lys Cys Gly Pro Thr
       ACT TCC GAA TAC TGT TGT ACC ACT GGT AAG TGT GGT CCA ACC

               170                                    180
       Glu Tyr Ser Arg Phe Phe Lys Arg Leu Cys Pro Asp Ala Phe
       GAA TAC TCT AGA TTC TTC AAG AGA TTG TGT CCA GAC GCT TTC

                                       190
       Ser Tyr Val Leu Asp Lys Pro Thr Thr Val Thr Cys Pro Gly
       TCC TAC GTC TTG GAC AAG CCA ACT ACC GTC ACT TGT CCA GGT

                       200
       Ser Ser Asn Tyr Arg Val Thr Phe Cys Pro Thr Ala
       TCT TCC AAC TAC AGA GTT ACC TTC TGT CCA ACT GCC-3'
```

8. A manufactured gene according to claim 1 wherein base codons specifying a polypeptide having one or more of the biochemical or immunological properties of thaumatin include initial and/or terminal codons respectively specifying additional initial and/or terminal amino acids in the polypeptide synthesized.

9. A manufactured gene according to claim 8 wherein said initial codons specifying additional initial amino acids are codons specifying an initial methione residue.

10. A manufactured gene according to claim 1 wherein the base codons specifying a polypeptide having the biological properties of thaumatin are preceded and/or followed by a sequence of bases comprising a portion of a base sequence which provides a recognition site for restriction endonuclease enzyme cleavage.

11. A manufactured gene according to claim 10 including the sequence of nucleotide bases set out in claim 5 preceded by the sequence 5'-GGATCCCG-3' and followed by the sequence 5'-TAATGACTCGAG-3'.

12. A fusion gene comprising a manufactured gene according to claim 1 fused to a second gene capable of directing synthesis of a second polypeptide in a manner permitting the synthesis of a fused polypeptide including the polypeptide product coded for by a manufactured gene of claim 1 and said second polypeptide.

13. A fusion gene according to claim 12 wherein said second gene is a gene directing synthesis of S.typhimurium L-ribulokinase enzyme.

14. A biologically functional DNA microorganism transformation vector including a manufactured gene according to claim 1.

15. A biologically functional DNA microorganism transformation vector including a fusion gene according to claim 12.

16. A vector according to either of claims 14 or 15 which is a circular DNA plasmid.

17. A vector according to claim 14 or 15 further including a regulatable selected promoter/regulator DNA sequence.

18. A vector according to claim 17 wherein said promoter/regulator DNA sequence is selected from among the group consisting of sequences duplicating the promoter/regulator sequences associated with yeast synthesis of 3-phosphoglycerate kinase or iso-1-cytochrome C or S.typhimurium synthesis of L-ribulokinase.

19. A vector according to claim 17 wherein said promoter/regulator DNA sequence is a tac promoter/regulator sequence.

20. A vector according to claim 14 or 15 further including a selected terminator DNA sequence.

21. A vector according to claim 20 wherein said terminator DNA sequence is selected from among the group consisting of sequences duplicating the terminator sequences associated with yeast synthesis of 3-phosphoglycerate kinase or iso-1-cytochrome C.

22. A microoganism transformed with a vector according to claim 14, 15, 16, 17 or 20.

23. A process for the production of a polypeptide having one or more of the biochemical or immunological properties of thaumatin comprising:

growing, under appropriate nutrient conditions, microorganisms transformed with a biologically functional DNA including a manufactured gene according to claim 1, whereby said microorganisms express said gene and produce said polypeptide.

24. A process according to claim 23 wherein the microorganisms grown are E.coli microorganisms.

25. A process according to claim 23 wherein the microorganisms grown are S.cerevisiae microorganisms.

26. A polypeptide product of the expression in a microorganism of a manufactured gene according to claim 1.

27. A polypeptide product of the expression in a microorganism of a manufactured gene according to claim 11.

28. An improved process for selectively altering the nucleotide sequence of a double-stranded plasmid DNA sequence having at least two unique restriction endonuclease enzyme recognition sites, wherein a single-stranded primer oligonucleotide containing the selected alteration is annealed to one strand of said double-stranded DNA sequence and extended to form a partial complement thereof, the improvement comprising the steps of:

(a) linearizing said double-stranded plasmid DNA sequence by restriction endonuclease enzyme digestion at a first unique recognition site in said sequence;

(b) denaturing the linearized double-stranded DNA sequence formed in step (a) into two complementary linear single-stranded DNA sequences;

(c) annealing said primer to one of said linear single-stranded sequences formed in step (b) and extending said primer to form a partially double-stranded DNA sequence;

(d) denaturing said partially double-stranded sequence formed in step (c) into the original plasmid-derived single-stranded sequence and a primer-derived single-stranded DNA sequence;

(e) linearizing said double-stranded plasmid DNA sequence by restriction endonuclease enzyme digestion at a second unique recognition site in said sequence;

(f) denaturing the linear sequence formed in step (e) into two complementary single-stranded DNA sequences;

(g) annealing the primer-derived single-stranded DNA sequence formed in step (d) to a plasmid-derived complementary single-stranded sequence formed in step (f);

(h) recircularizing the annealed strands of step (g) into a double-stranded DNA plasmid with an alteration in one strand;

(i) transforming a host microorganism with the plasmid formed in step (h) and isolating daughter cell populations containing plasmids with the selectively altered sequence by hybridization with said primer; and

(j) transforming a host microorganism with the selectively altered plasmids obtained from the hybridization of step (i).

29. An improved process for selectively altering the nucleotide sequence of a double-stranded plasmid DNA sequence having first, second and third unique restriction endonuclease enzyme recognition sites, wherein a single-stranded primer oligonucleotide containing the selected alteration is annealed to one strand of said double-stranded DNA sequence and extended to form a partial complement thereof, and the plasmid DNA sequence to be altered is between said second and third recognition sites, the improvement comprising the steps of:

(a) linearizing said double-stranded plasmid DNA sequence by restriction endonuclease enzyme digestion at said first recognition site in said sequence;

(b) denaturing the linearized double-stranded DNA sequence formed in step (a) into two complementary linear single-stranded DNA sequences;

(c) annealing said primer to one of said linear single-stranded sequences formed in step (b) and annealing to the same linear sequence a second primer complementary to a portion of said sequence between said first and second recognition sites and extending said primers to form a partially double-stranded DNA sequence including both said second and third recognition sites;

(d) cleaving said partially double-stranded sequence formed in step (c) by restriction endonuclease digestion at said second and third restriction sites to form a double-stranded sequence which is a hybrid of a plasmid single strand and said primer extended strands;

(e) inserting said fragment formed in step (d) into said DNA plasmid to form a double-stranded plasmid including said alteration in one of its strands;

(f) transforming a host microorganism with the plasmid formed in step (e) and isolating daughter cell populations containing double-stranded plasmids with the selectively altered sequence by hybridization with said primer; and

(g) transforming a host microorganism with the selectively altered plasmids obtained from the hybridization of step (f).